# EUROPÄISCHE PATENTSCHRIFT

(11) **EP 0 659 172 B1**
(45) Veröffentlichungstag und Bekanntmachung des Hinweises auf die Patenterteilung: **02.04.1997**
(21) Anmeldenummer: 93918997.3
(22) Anmeldetag: 10.09.1993
(51) Int. Cl.: C07C 53/128, C07C 53/132, C07C 57/03, C07C 57/18, C07C 51/00, A61K 31/19

(54) **VPA-ANALOGE ANTIEPILEPTIKA**
ANALOGUES OF VPA USED AS ANTI-EPILEPTICA
ANTIEPILEPTIQUES ANALOGUES DU VPA

(30) Priorität: 12.09.1992 DE 4231085
(43) Veröffentlichungstag der Anmeldung: 28.06.1995
(73) Patentinhaber: NAU, Heinz, D-14163 Berlin (DE)
(72) Erfinder: NAU, Heinz, D-14163 Berlin (DE); BOJIC, Ursula, D-12165 Berlin (DE); HAUCK, Ralf-Siegbert, D-12169 Berlin (DE); ELMAZAR, Mohamed Mohey Eldin, Heliopolis, Cairo (EG)
(74) Vertreter: Wablat, Wolfgang, Dr.Dr.
(86) Internationale Anmeldenummer: DE9300861
(87) Internationale Veröffentlichungsnummer: WO9406743

(56) Entgegenhaltungen:
- EP-A- 0 293 752
- DE-A- 2 146 417
- DE-A- 2 854 195
- US-A- 3 325 361

## Beschreibung

Die Erfindung betrifft Verbindungen, die Analoge der Valproinsäure sind, ihre Verwendung als Antiepileptikum und ihre Herstellung.

Epilepsie ist eine chronische Krankheit, an der etwa 0,5 % bis 2 % der Bevölkerung leidet. Als Antiepileptikum ist beispielsweise Walproinsäure (2-n-Propylpentansäure) vorgeschlagen worden (US-A-3 325 361). Neben der antiepileptischen (antikonvulsiven) Wirkung kann Valproinsäure jedoch zu teratogenen und sedierenden (neurotoxischen) Effekten führend. Fast 1 % aller schwangeren Frauen leiden an Epilepsie. Das Mißbildungsrisiko für Kinder von Müttern mit Epilepsie ist um das 1½- bis 3-fache höher als bei gesunden Müttern. Die gesteigerte Inzidenz der Fehlbildungen ist wahrscheinlich multifaktoriell bedingt, wobei neben den Antiepileptika auch genetische Faktoren sowie Form und Schwere der Epilepsie eine Rolle spielen können. Auch bei Valproinsäure hat sich erwiesen, daß vermehrt Kinder mit Mißbildungen zur Welt kamen.

Es hat daher nicht an Versuchen gefehlt, weitere gleich stark oder stärker antiepileptisch wirkende Verbindungen zu finden, die eine geringere Teratogenität als Valproinsäure aufweisen und nur gering sedativ wirken. So sind beispielsweise 2-n-Propyl-2-pentensäure, 2-n-Propyl-3-pentensäure, 2-n-Propyl-4-pentensäure und 2-n-Propyl-4-pentinsäure getestet worden. Die antiepileptische Wirkung dieser Substanzen ist jedoch geringer als die der Valproinsäure, die letzteren der erwähnten Substanzen sind im Tierversuch auch teratogen.

Der Erfindung liegt daher die Aufgabe zugrunde, Verbindungen zur Verfügung zu stellen, die wirksame Antiepileptika sind und dabei nur geringe sedierende und teratogene Wirkung zeigen.

Diese Aufgabe wird durch Verbindungen mit der Formel 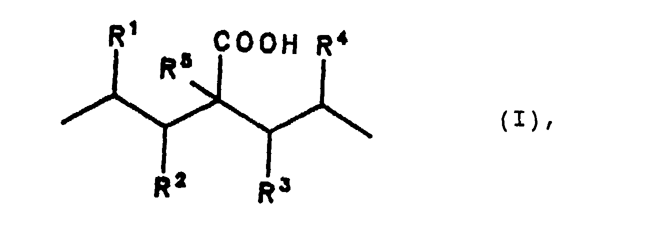 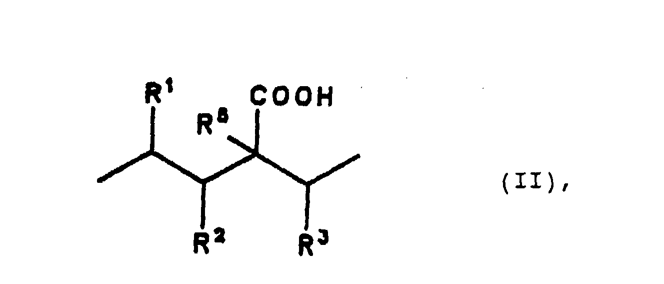 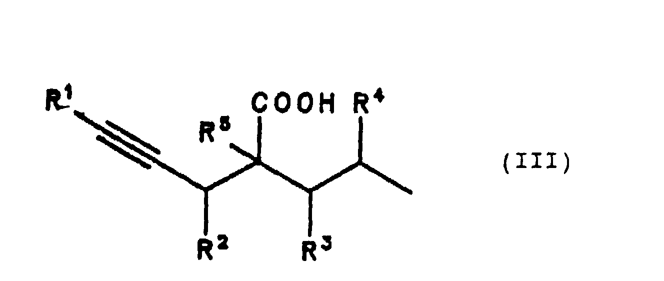 oder 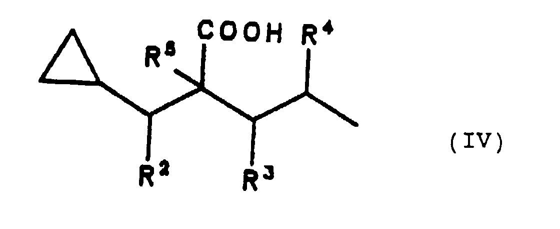 wobei die Verbindungen der Formel (I) und (II) auch ein- oder mehrfach ungesättigt sein können, in denen R¹, R², R³ und R⁴ unabhängig voneinander Wasserstoff oder eine C₁-C₆-Alkylgruppe sind, R⁵ Wasserstoff oder eine C₁-C₂-Alkylgruppe ist und bei den Verbindungen der Formeln (I) und (II) mindestens einer der Reste R¹ bis R⁴ von Wasserstoff verschieden ist, wobei die Verbindungen der Formel (I), wenn am Kohlenstoffatom in der 2-Position ein Asymmetriezentrum vorhanden ist, in Form ihres Racemats, ihrer reinen Enantiomere oder eines vom Racemat verschiedenen Gemisches ihrer Enantiomere vorliegen und die Verbindungen der Formeln (II), (III) und (IV) in Form ihrer reinen Enantiomere oder eines vom Racemat verschiedenen Gemisches ihrer Enantiomere vorliegen, wobei die Verbindungen der Formel (III), wenn sie an mindestens an einer anderen als der 5-Position alkyliert sind, und die Verbindungen der Formel (IV), wenn sie mindestens einfach alkyliert sind, auch in Form ihres Racemats vorliegen, sowie pharmazeutisch verträgliche Salze derselben.

Das erfindungsgemäße Antiepileptikum umfaßt eine Verbindung der Formel 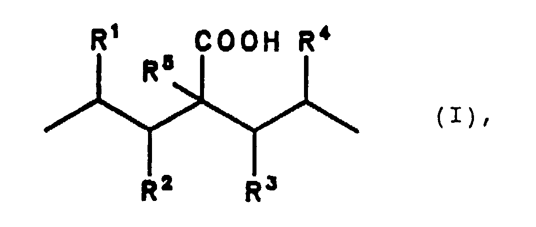 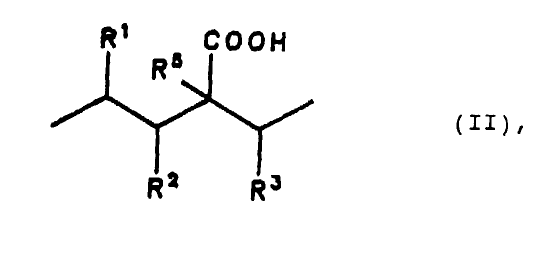 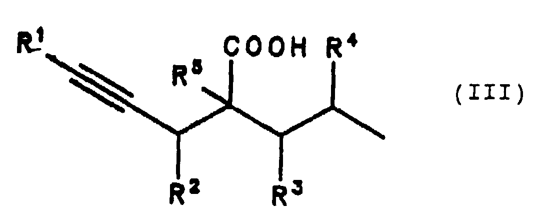 oder 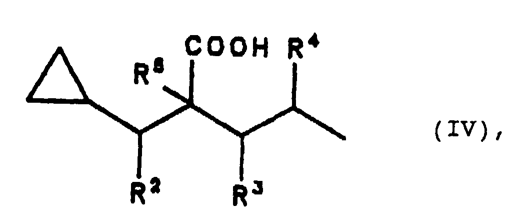 wobei die Verbindungen der Formeln (I) und (II) auch ein- oder mehrfach ungesättigt sein können, umfaßt, in denen R¹, R², R³ und R⁴ jeweils unabhängig voneinander Wasserstoff oder eine C₁-C₆-Alkylgruppe sind, R⁵ Wasserstoff oder eine C₁-C₂-Alkylgruppe ist und bei den Verbindungen der Formeln (I) und (II) mindestens einer der Reste R¹ bis R⁴ von Wasserstoff verschieden ist, wobei die Verbindung der Formel (I) für den Fall, daß am Kohlenstoffatom in der 2-Position ein Asymmetriezentrum vorhanden ist, und die Verbindung der Formeln (II), (III) und (IV) in Form ihres Racemats, ihrer reinen Enantiomere oder eines vom Racemat verschiedenen Gemisches ihrer Enantiomere vorliegen, oder pharmazeutisch verträgliche Salze derselben.

Bevorzugte Ausführungsformen sind Gegenstand der Unteransprüche.

Überraschenderweise hat sich gezeigt, daß eine zusätzliche Substitution des Valproinsäuregerüstes bzw. eines Homologen desselben (mit kürzeren oder längeren Seitenketten am Kohlenstoffatom in der 2-Position) in den Positionen 5, 4, 3, 2, 2', 3' und/oder 4' durch eine C₁-C₆-Alkylgruppe eine erhebliche Senkung der teratogenen Wirkung mit sich bringt.

Besonders vorteilhaft ist dieser Effekt, wenn es sich bei dem mindestens einen, die an das C-2-Atom gebundenen Seitenketten verzweigenden Substituenten um eine Methyl- oder Ethylgruppe und insbesondere eine Methylgruppe handelt. Besonders bevorzugt sind hierbei Verbindungen mit den Formeln 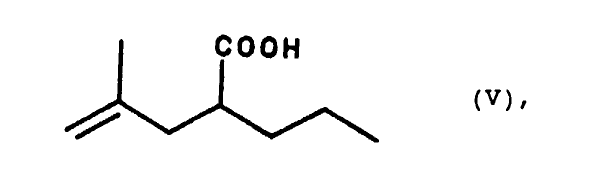 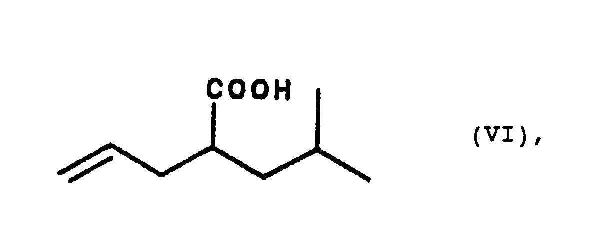 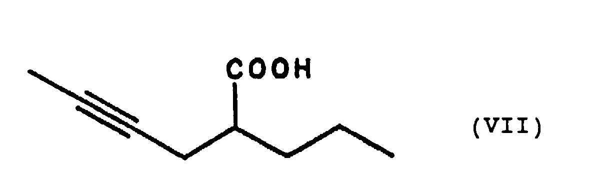 und

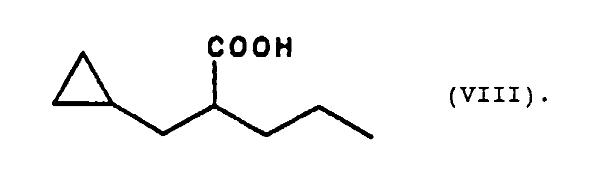

Neben der Verzweigung der Seitenketten ist daher bei den Verbindungen (I) und (II) vorzugsweise am Kohlenstoffatom in der 4-Position und/oder der 2'-Position ferner eine ungesättigte C-C-Bindung, insbesondere eine Doppelbindung vorhanden.

Für den Fall, daß das Kohlenstoffatom in der 2-Position ein Asymmetriezentrum ist, sind die Verbindungen sowohl in Form ihres Racemats als auch ihrer reinen Enantiomere oder eines vom Racemat verschiedenen Gemisches ihrer Enantiomere antiepileptisch wirksam, wobei die Teratogenität in jedem dieser Fälle gering ist. Besonders gering ist die Teratogenität bei den jeweiligen R-Enantiomeren.

Außer den freien Carbonsäuren können auch deren pharmazeutisch verträglichen Salze wie die Alkali-, Erdalkali- oder Ammoniumsalze eingesetzt werden. Der antiepileptische bzw. antikonvulsive Effekt ist unabhängig davon, ob das jeweilige R- oder S-Enantiomer oder ein Enantiomerengemisch vorliegt, d.h. er ist von der diesbzüglichen Stereochemie unabhängig (nicht stereoselektiv).

Neben der verringerten teratogenen Wirkung ist auch die sedative Wirkung der erfindungsgemäßen Verbindungen gegenüber derjenigen der Valproinsäure erheblich verringert und konnte teilweise nicht festgestellt werden.

Die erfindungsgemäßen Verbindungen sind daher ausgezeichnet als Antiepileptika geeignet.

Die Synthese archiraler und racemischer Carbonsäuren erfolgt in Anlehnung an die allgemein bekannte Malonestersynthese, indem ein 2-Alkylmalonsäuredialkylester, z.B. der Diethylester, mit der Formel 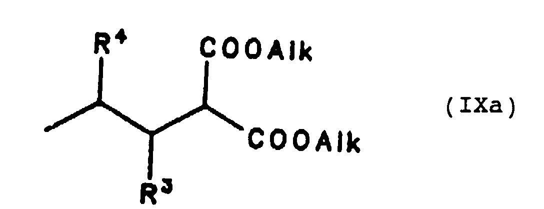 oder 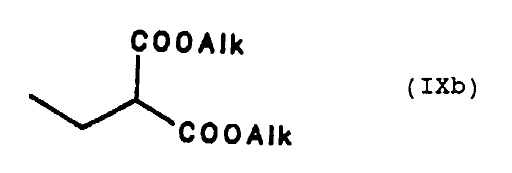 Alk = Methyl, Ethyl
mit einer Base wie beispielsweise Natriumethylat deprotoniert und mit einem Alkylierungsmittel der Formel 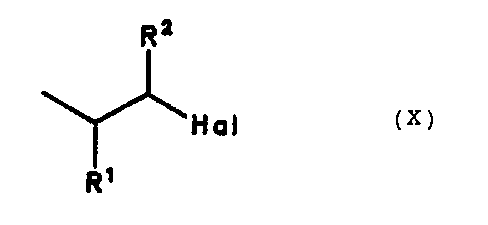 am Kohlenstoff in der 2-Position alkyliert wird. Der dialkylierte Malonsäuredialkylester der Formel 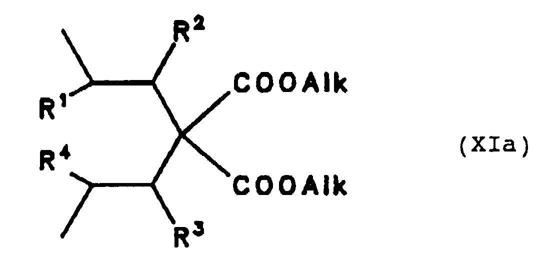 oder 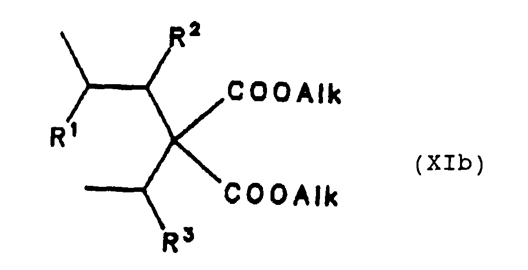 wird anschließend alkalisch, z.B. mit Kaliumhydroxid in einem Wasser/Ethanol-Gemisch, verseift und unter Erhitzen decarboxyliert, um die gewünschte Carbonsäure zu liefern. In Abhängigkeit von der eventuell in dem gewünschten Produkt vorhandenen Ungesättigtheit, können der als Ausgangsverbindung eingesetzte Malonester oder das Alkylierungsmittel an den betreffenden Positionen auch ungesättigt sein.

### Allgemeine Synthesevorschrift:

3,1 g (135 mMol) Natrium werden in 100 ml absolutem Ethanol gelöst und mit 25,6 ml (125 mMol) 2-n-Alkylmalonsäurediethylester in 50 ml absolutem Ethanol versetzt. Anschließend werden 150 mMol frisch destilliertes Alkylhalogenid (RX, siehe die genauen Bezeichnungen bei der Beschreibung der einzelnen Verbindungen) so zugetropft, daß die Reaktionsmischung gerade siedet und bis zum Reaktionsende unter Rückfluß gekocht (ca. 4 bis 12 Stunden). Ethanol wird im Wasserstrahlvakuum entfernt; ausgefallenes Natriumhalogenid wird in 150 ml Wasser aufgenommen, mit verdünnter HCl angesäuert (pH < 2) und mit Diethylether ausgeschüttelt (3 x 200 ml). Die organische Phase wird über wasserfreiem Natriumsulfat getrocknet und im Wassertrahlvakuum abgezogen. Der zurückbleibende dialkylierte Malonester wird durch fraktionierte Destillation im Hochvakuum gereinigt und zu einer Lösung von 20,3 g (350 mMol) Kaliumhydroxid in 50 ml Wasser und 100 ml Ethanol gegeben. Die Reaktionsmischung wird bis zur vollständigen Verseifung unter Rückfluß zum Sieden erhitzt (12 Stunden). Ethanol wird im Wasserstrahlvakuum abgezogen, der wäßrige Rückstand mit 200 ml Wasser versetzt und mit Diethylether ausgeschüttelt (3 x 300 ml). Die organische Phase wird verworfen, die wäßrige wird unter Rühren, tropfenweise mit konzentrierter HCl angesäuert (pH < 2) und mit Diethylether ausgeschüttelt (3 x 300 ml). Die organische Phase wird über Natriumsulfat getrocknet und im Wasserstrahlvakuum eingeengt. Die rohe dialkylierte Malonsäure wird ohne weitere Reinigung im Ölbad auf 160 bis 180°C erhitzt und nach beendeter Decarboxylierung (4 Stunden) 2 mal im Hochvakuum destilliert.

Die chromatographischen und massenspektroskopischen Untersuchungen wurden wie folgt durchgeführt:
a) Chromatographieverfahren
   Für die Dünnschichtchromatographie wurden Kieselgelplatten mit Fluoreszenzindikator der Firma Merck (Kieselgel 60 F₂₅₄, 0,2 mm Schichtdicke) verwendet. Neben der UV-Detektion wurde zur Sichtbarmachung von Substanzflecken die Kieselgelplatten mit halbkonzentrieter Schwefelsäure besprüht und auf ca. 150°C erwärmt. Als Laufmittel dienten Gemische aus n-Hexan und Essigsäureethylester (V/V). Bei der Untersuchung der α-verzzweigten Carbonsäuren wurde das folgende Laufmittelgemisch verwendet: 75 ml n-Hexan + 20 ml Essigsäureethylester + 5 ml Essigsäure. Die präparative Säulenchromatographie wurde an Kieselgel 60 (0,040 - 0,063 mm, 230 - 400 mesh ASTM) der Firma Merck unter Anwendung der Flash-Technik (Still et al. J. Org. Chem. **43**, 2923 - 2925 (1978)) durchgeführt.
b) Massenspektroskopie (GC-MS-Kopplung)
   Die Massenspektren der TMS-Carbonsäuren wurden mit Hilfe einer GC-MS-Kopplung der Firma Hewlett Packard erhalten: (a) Der GC, Typ HP 5890 A, war mit einer HP-1 Kapillarsäule (12 m Länge x 0,2 mm Innendurchmesser x 0,33 µm Schichtdicke) bestückt. Helium diente als Trägergas, der Gasvolumenfluß betrug 0,5 ml/min. Die Temperatur des Injektors war 250°C, sie initiale Säulentemperatur, die für 1 Minute konstant gehalten wurde, 70°C. Die Temperatur wurde kontinuierlich auf 170°C erhöht, je 10°C/min und 1 Minute konstant gehalten. (b) Das Massenspektrometer, Typ 5971 MSD, wurde im Scan-Sensivity-Electron Impact Ionization Modus zwischen den Massen (m/z) 60 und 270 betrieben. Die Ionisierungsenergie betrug 70 eV. Die Ionenquellentemperatur war 180°C und das GC-Interface wurde auf 280°C gehalten. Das MSD Datensystem, Hewlett Packard G 1030 A Chem Station, diente zur Aufnahme der Spektren. Die relativen prozentualen Intensitäten der Ionen wurden auf das Trimethylsilylanion (m/z = 73) bezogen. Die Carbonsäuren wurden in Methylenchlorid gelöst und durch Zugabe von MSTFA in die TMS-Carbonsäureester überführt (Raumtemperatur, 2h).

### Beispiel 1

### R,S-4-Methyl-2-n-propyl-4-pentensäure (V)

Die Synthese erfolgt nach der oben angegebenen allgemeine Vorschrift, wobei 2-n-Propylmalonsäurediethylester und 3-Chlor-2-methylpropen als Ausgangsverbindung bzw. Alkylierungsmittel eingesetzt werden. Die Verbindung wird säulenchromatographisch an Kieselgel 60 (Elutionsmittel: n-Hexan/Essigsäureethylester/Essigsäure = 94/3/3) gereinigt.

| | |
|---|---|
| Ausbeute: | 8,0 g (41 %) |
| Siedepunkt: | 70 bis 71°C/0,1 mbar. |
| DC: | R_{f} = 0,57. |

¹H-NMR (270MHz/CDCl₃): δ = 0,88 (t, J = 7 Hz, 3H, 3'H), 1,20-1,65 (m, 4H, 1'H, 2'-H), 1,70 (s. 3H, 4-CH₃), 2,07-2,20, 2,28-2,40 (2m, 2H, 3-HₐH_{b}), 2,48-2,64 (m, 1H, 2-H), 4,72 (d, J = 11 Hz, 2H 5-H), 11,48 (s. (breit), 1H, COOH].
¹³C-NMR (270MHz/CDCl₃): δ = 13,87, 20,45, 22,16, 34,08, 40,34, 43,67, 44,53, 112,23, 142,64, 182,63.
GC-MS (TMS-Ester): Retentionszeit = 5,96 Minuten;
m/z = 213 (18 %, M⁺-CH₃), 199 (4 %, M⁺-C₂H₅), 186 (11 %, M⁺-C₃H₆).

### Beispiel 2

### R,S-2-n-Propyl-4-hexinsäure (VII)

Die Synthese erfolgt nach der oben angegebenen allgemeinen Syn-thesevorschrift, wobei 2-n-Propylmalonsäurediethylester und 1-Brom-2-butin als Ausgangsverbindung bzw. Alkylierungsmittel eingesetzt werden. 1-Brom-2-butin wurde nach der von K.E. Schulte und Reiss in Chemische Berichte **87**, 964-970 (1954) beschriebenen Methode synthetisiert.

| | |
|---|---|
| Ausbeute: | 6,9 g (36 %) |
| Siedepunkt: | 94°C/0,2 mbar |
| DC: | R_{f} = 0,46 |

¹H-NMR (270MHz/CDCl₃): δ = 0,93 (t, J = 7,5 Hz, 3H, 3'-H), 1,28-1,47 (m, 2H, 2'-H), 1,54-1,73 (m, 2H, 1'-H), 1,76 (t, J = 2 Hz, 3H, 6-H), 2,27-2,61 (m, 3H, 2-H, 3-H), 11,45 [s. (breit), 1H, COOH].
¹³C-NMR (270MHz/CDCl₃): δ = 3,32, 13,80, 20,21, 21,18, 33,18, 44,68, 75,80, 76,53, 181,55.
GC-MS (TMS-Ester): Retentionszeit = 7,17 Minuten;
m/z = 211 (60 %, M⁺-CH₃), 184 (24 %, M⁺-C₃H₆), 183 (122 %, M⁺-C₃H₇).

### Beispiel 3

### R,S-2-(Cyclopropylmethyl)-pentansäure (VIII)

Die Synthese erfolgt nach dem oben angegebenen allgemeinen Syntheseverfahren, wobei 2-n-Propylmalonsäurediethylester und Chlormethylcyclopropan als Ausgangsverbindung bzw. Alkylierungsmittel eingesetzt werden.

| | |
|---|---|
| Ausbeute: | 9,4 g (48 %) |
| Siedepunkt: | 91-93°C/1,2 mbar |
| DC: | R_{f} = 0,58 |

¹H-NMR (270MHz/CDCl₃): δ = -0,08-0,12 (m, 2H, C₃H₅-Hₜᵣₐₙₛ), 0,28-0,48 (m, 2H, C₃H₅-H_{cis}), 0,61-0,76 (m, 1H, C₃H₅-Hₐ), 0,89 (t, J = 8 Hz, 3H, 5-H), 1,21-1,68 (m, 6H, 3-H, 4-H, C₃H₅CH₂), 2,46 (mc, 1H, 2-H), 12,04 [s (breit), 1H, COOH]. Mit Hₐ wird das Proton am tertiären Kohlenstoff des Cyclopropanringes bezeichnet. Die anderen Ring-Protonen sind zu Hₐ cis- bzw. trans-ständig angeordnet (H_{cis} bzw. Hₜᵣₐₙₛ).
¹³C-NMR (170MHz/CDCl₃): δ= 4,31, 4,49, 8,99, 13,93, 20,53, 34,17, 37,24, 45,90, 183,52.
GC-MS (TMS-Ester): Retentionszeit = 6,74 Minuten;
m/z = 213 (30 %, M⁺-CH₃), 199 (89 %, M⁺-C₂H₅), 186 (13 %, M⁺-C₃H₆).

### Beispiel 4 (Vergleichsbeispiel)

### R,S-2-n-Propyl-4-pentinsäure (XII)

Die Synthese erfolgt nach der oben angegebenen allgemeinen Synthesevorschrift, wobei 2-n-Propylmalonsäurediethylester und Propargylchlorid als Ausgangsverbindung bzw. Alkylierungsmittel eingesetzt werden.

| | |
|---|---|
| Ausbeute: | 7,7 g (44 %) |
| Siedepunkt: | 75-76°C/0,7 mbar |
| Lit.: | 82-83°C/2 Torr [Cerbai et al. Farmaco, Ed. Sci, **27**, 217 -234 (1971)]. |
| DC: | R_{f} = 0,40 |

¹H-NMR (270MHz/CDCl₃): δ = 0,94 (t, J = 8 Hz, 3H, 3'-H), 1,28-1,50 (m, 2H, 2'-H), 1,57-1,81 (m, 2H, 1'-H), 2,02 (t, J = 2,5 Hz, 5-H), 2,35-2,53 (m, 2H, 3-H), 2,54-2,70 (m, 1H, 2-H), 12,00 (s. 1H, COOH).
¹³C-NMR (270Mz/CDCl₃): δ = 13,71, 19,95, 20,65, 33,02, 44,05, 69,92, 81,06, 181.10.
GC-MS (TMS-Ester): Retentionszeit: 5,31 Minuten;
m/z = 197 (32 %, M⁺-CH₃), 170 (20 %, M⁺-C₃H₆), 169 (13 %, M⁺-C₃H₇).

### Beispiel 5 (Vergleichsbeispiel)

### R,S-2-n-Propyl-4-pentensäure (XIII)

Die Synthese erfolgt nach der oben angegebenen allgemeinen Synthesevorschrift, wobei 2-n-Propylmalonsäurediethylester und Allylbromid als Ausgangsverbindung bzw. Alkylierungsmittel eingesetzt werden.

| | |
|---|---|
| Ausbeute: | 8,7 g (49 %) |
| Siedepunkt: | 87-88°C/2,4 mbar |
| DC: | R_{f} = 0,65 |

¹H-NMR (270MHz/CDCl₃): δ = 0,91, (t, J = 8 Hz, 3H, 3'-H), 1,30-1,73 (m, 4H, 1'-H, 2'-H), 2,20-2,54 (m, 3H, 2-H, 3-H), 5,02-5,15 (m, 2H, 5-H,), 5,80 (mc, 1H, 4-H), 12,05 (s. 1H, COOH).
¹³C-NMR (270MHz/CDCl₃): δ = 13,84, 20,36, 33,63, 36,07, 45,02, 116,84, 135,18, 182,51.
GC-MS (TMS-Ester): Retentionszeit = 5,01 Minuten;
m/z = 199 (37 %, M⁺-CH₃), 185 (14 %, M⁺-C₂H₅), 172 (29 %, M⁺-C₃H₆).

### Beispiel 6 (Vergleichsbeispiel)

### 2-Methyl-2-propylpentansäure (XIV)

11,0 ml (70 mMol) 2-n-Propylpentansäure werden zu 150 mMol LDA-Lösung (hergestellt aus 21,0 ml Diisopropylamin in 100 ml THF und 90 ml n-BuLi (1,6 M in n-Hexan)) hinzugetropft. Die Reaktionsmischung wird auf 50°C erhitzt und kräftig gerührt. Nach beendeter Metallierung wird auf Raumtemperatur abgekühlt, 4,4 ml (70 mMol) Methyljodid hinzugegeben und 2 Stunden gerührt. Nach dem Ansäuern mit 3 molarer HCl (200 ml) wird mit n-Hexan ausgeschüttelt (3 x 200 ml). Die vereinigten organischen Phasen werden über Natriumsulfat getrocknet und eingeengt. Die nach Destillation im Hochvakuum anfallende kristalline Verbindung wird aus n-Hexan umkristallisiert.

| | |
|---|---|
| Ausbeute: | 3,5 g (32%) |
| Siedepunkt: | 71°C/0,05 mbar |
| DC: | R_{f} = 0,62. |

¹H-NMR (270MHz/CDCl₃): δ = 0,91, (t, J = 7 Hz, 6H, 5-H, 3'-H), 1,15 (s, 3H,2-CH₃) 1,19-1,44 und 1,56-1,68 (m, 8H, 3-H, 4-H, 1'-H, 2'-H), 12,12 (s, breit), 1H, COOH).
¹³C-NMR (270MHz/CDCl₃): δ = 14,57, 17,76, 20,95, 41,48, 45,89, 184,94.
GC-MS (TMS-Ester): Retentionszeit = 9,07 Minuten;
m/z = 215 (36%, M⁺-CH₃), 188 (22%, M⁺-C₃H₆).

Die Alkylierung der Carbonsäuren an der 2-Position mit Alkylhalogeniden ist ganz allgemein mit Hilfe von n-Butyllithium bei erhöhter Temperatur möglich (Pfeffer et al., Alpha-anions of carboxylic acids. II. The formation and alkylation of alpha-metalated aliphatic acids. Journal of Organic Chemistry **37**, 451-458 (1972)). Die Methylierung des tertiären Kohlenstoffatoms der Valproinsäure gelingt beispielsweise durch Deprotonierung des α-Wasserstoffatoms am C-2 unter Verwendung von n-Butyllithium in Hexan bei 50°C und anschließende Methylierung mit Methyljodid bei Raumtemperatur in 32 %-iger Ausbeute. Entsprechend diesem Verfahren können auch die übrigen 2-alkylierten Carbonsäuren hergestellt werden.

### Synthese enantiomerer Carbonsäuren

Zur Synthese enantiomerer Carbonsäuren können chirale Auxiliare wie RAMP und SAMP oder Oxazolidinon-Auxiliare verwendet werden.

Die Synthese der Enantiomeren der 2-n-Propyl-4-hexinsäure mit Hilfe chiraler Oxazolidinon-Auxiliare basiert auf der asymmetrischen Alkylierung von chiralen Imid-Enolaten, die 1982 von Evans et al. beschrieben wurde (J. Am. Chem. Soc. **104**, 1737-1739 dienen beispielsweise die im Handel erhältlichen Oxazolidinone (XV) und (XVI).

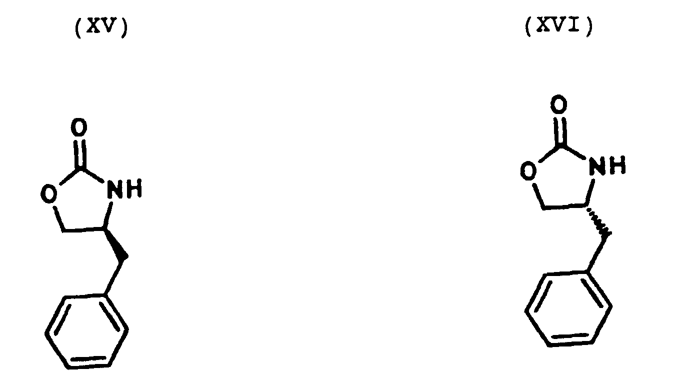

### Beispiel 7

### Synthese von R-2-n-Propyl-4-hexinsäure

a) S-4-Benzyl-3-(1-oxopentyl)-2-oxazolidinon (XVII)
   Das Alkylierungsmittel 1-Brom-2-butin wurde analog der von Schulte und Reiss in Chem. Berichte **87**, 964 - 979 (1954) beschriebenen Methode dargestellt. 250 mMol Oxazolidinon in 330 ml absolutem THF werden bei -78°C, tropfenweise mit 156,0 ml (255 mMol) n-BuLi (1,6 M in n-Hexan) versetzt (die orange-rote Farbe des Dianions muß gerade erhalten bleiben). Nach 1 Stunde Rühren werden 30,7 ml (255 mmol) frisch destilliertes Valeroylchlorid hinzugetropft. Die Reaktionsmischung wird langsam auf Raumtemperatur erwärmt und weitere 4 Stunden gerührt. Nach beendeter Acylierung wird die Reaktion durch Hinzugabe halbgesättigter Ammoniumchloridlösung (200 ml) abgebrochen. Im Wasserstrahlvakuum werden die flüchtigen Bestandteile abgezogen und der Rückstand mit Methylenchlorid extrahiert (3 x 200 ml). Die vereinigten organischen Phasen werden zunächst mit Wasser, dann mit Natriumchloridlösung gewaschen, über Na₂SO₄ getrocknet und eingeengt. Das gelbe, kristalline N-acylierte Oxazolidinon-Rohprodukt wird durch Umkristallisieren in n-Pentan gereinigt.
   [α]²²_{D} = 53,2 (c = 2,4, Chloroform)
   Ausbeute: 65,3 g (96%)
   ¹H-NMR (270MHz/CDCl₃): δ = 0,95, (t, J = 6,5 Hz, 3H, 5'-H), 1,42 (dt, J₁ = 8Hz, J₂ = 6,5 Hz, 2H, 4'-H), 1,60-1,75 (m, 2H, 3'-H), 2,77 (dd, J₁ = 13,5 Hz, J₂ = 9 Hz, 1H, C₆H₅CH_{b}), 2,83 - 3,05 (m, 2H, 2'-H), 3,30 (dd, J₁ = 13,5 Hz, J₂ = 3Hz, 1H, C₆H₅CHₐ), 4,12 - 4,24 (m, 2H, 5-H), 4,63 - 4,73 (m, 1H, 4-H), 7,18 - 7,37 (m, 5H, C₆H₅).
b) (4S,2'R)-4-Benzyl-3-(1-oxo-2-n-propyl-4-hexinyl)-2-oxazolidinon
   Die Synthese des herzustellenden alkylierten Oxazolidnons erfolgt, indem 65 mMol N-acyliertes Oxazolidinon in 25 ml absolutem THF bei -78°C tropfenweise zu 70 mMol einer frisch bereiteten LDA-Lösung (hergestellt aus 9,8 ml Diisopropylamin in 85 ml absolutem THF und 43,8 ml n-BuLi (1,6 M in n-Hexan)) hinzugegeben. Nach 30 Minuten Rühren wird das Lithium-Enolat schnell mit 80 mMol 1-Brom-2-butin versetzt und die Reaktionsmischung in sehr kurzer Zeit auf -20°C und dann innerhalb von 6 Stunden auf 10°C erwärmt. Die Reaktion wird durch Hinzugabe halbgesättigter Ammoniumchloridlösung abgebrochen (200 ml). Die flüchtigen Bestandteile werden im Wasserstrahlvakuum abgezogen, der Rückstand angesäuert (1 molarer HCl) und mit Methylenchlorid extrahiert (3 x 200 ml). Die vereinigten organischen Phasen werden zunächst mit Wasser, dann mit gesättigter Natriumchloridlösung gewaschen, über Natriumsulfat getrocknet und eingeengt. Das gelbe, ölige Alkylierungsprodukt wird säulenchromatographisch gereinigt (Elutionsmittel: n-Hexan/Essigsäureethylester = 95/5).
   Ausbeute: 17,6 g (73 %).
   [α]_{D}²⁴ = + 68,6 (c = 2,2, Chloroform)
   ¹H-NMR (270MHz/CDCl₃): δ = 0,86-0,99 (m, 3H, 3"-H), 1,25-1,46 (m, 2H, 2'-H), 1,48-1,63 (m, 1H, 1"-Hₐ), 1,65- ≈ 1,82 (m, 1H, 1"-H_{b}), 1,76 (t, J = 3 Hz, 3H, 6'-H), 2,49 (mc, 2H, 3'-H), 2,77 (dd, J₁ = 13,5 Hz, J₂ = 9,5 Hz, 1H, C₆H₅CHₐ), 3,29 (dd, J₁ = 13,5 Hz, J₂ = 3Hz, 1H, C₆H₅CH_{b}) 4,96 (dt, J₁ = J₂ = 7 Hz, 1H, 2'-H), 4,12-4,25 (m, 2H, 5-H), 4,67-4,77 (m, 1H 4-H), 7,19-7,38 (m, 5H, C₆H₅). Das Multiplett von 1"-H wird vom Triplett der 6'-Protonen teilweise überdeckt.
c) R-2-n-Propyl-4-hexinsäure (R-VII)
   Zu einer auf 5°C gekühlten Lösung von 30 mMol Alkylierungsprodukt aus b) in 400 ml THF/Wasser (3:1) werden zunächst 23,2 ml (136 mMol) Wasserstoffperoxid (30 %-ig in Wasser) und anschließend 2,5 g (60 mMol) Lithiumhydroxid-Monohydrat in 65 ml Wasser hinzugetropft. Nach 6 Stunden Rühren ist die Reaktion beendet und wird durch Hinzutropfen einer Lösung von 28,4 g (225 mMol) Natriumsulfit in 120 ml Wasser unterbrochen. THF wird im Wasserstrahlvakuum abgezogen und die zurückbleibende wäßrige Phase mit 5 molarer Natronlauge alkylisiert (pH > 10). Das Oxazolidinon wird durch Ausschütteln mit Methylenchlorid (3 x 200 ml) extrahiert und aus der organischen Phase zurückgewonnen. Die wäßrige Phase wird mit 1 molarer HCl angesäuert (pH > 2) und mit Diethylether ausgeschüttelt (3 x 200 ml). Die vereinigten Etherphasen werden über Na₂SO₄ getrocknet und eingeengt. Die Carbonsäure wird durch zweimalige Destillation im Hochvakuum gereinigt.
   Ausbeute: 4,1 g (88 %)
   [α]_{D}²⁵ = -2,2 (c = 2,3, Chloroform);
   Enantiomerenüberschuß = 96 %
   Die ¹H-NMR (270MHz/CDCl₃)- und ¹³C-NMR (270MHz/CDCl₃)-Spektren sind identisch mit denen der racemischen Verbindung VII.

### Beispiel 8

### S-2-n-Propyl-4-hexinsäure (S-VII)

a) R-4-Benzyl-3-(1-oxopentyl)-2-oxazolidinon (XIX)
   Ausgehend von Verbindung (XVI) wird nach dem oben unter Beispiel 7 beschriebenen Verfahren R-4-Benzyl-3-(1-oxopentyl)-2-oxazolidinon in einer Ausbeute von 63,4 g (97 %) erhalten.
   [α]²⁴_{D} = 52,5 (c = 2,0, Chloroform)
b) (4R,2'S)-4-Benzyl-3-(1-oxo-2-n-propyl-4-hexinyl)-2-oxazolidinon (XX)
   Die Synthese erfolgt wie im vorherigen Beispiel beschrieben.
   Ausbeute: 18,4 g (76 %)
   [α]²²_{D} = 67,2 (c = 2,0, Chloroform)
c) S-2-n-Propyl-4-hexinsäure (S-VII)
   Die Synthese erfolgt wie im vorhergehenden Beispiel beschrieben.
   Ausbeute: 4,0 g (87%)
   [α]_{D}²⁵ = + 2,1 (c = 2,2, Chloroform)
   Enantiomerenüberschuß = 94 %
   Die ¹H-NMR (270MHz/CDCl₃)- und ¹³C-NMR (270MHz/CDCl₃)-Spektren sind identisch mit denen der racemischen Verbindung VII.

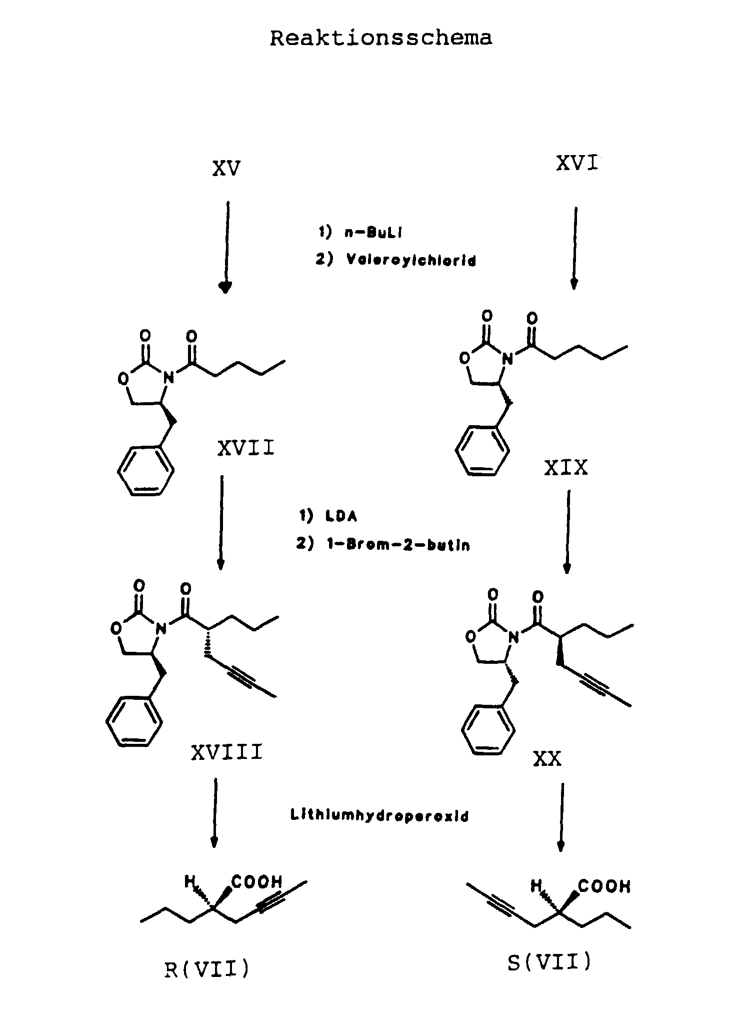

Die Enantiomere der übrigen erfindungsgemäßen Verbindungen können ebenfalls gemäß dem oben beschriebenen Verfahren hergestellt werden, wobei dann lediglich die jeweiligen Reagenzien entsprechend dem gewünschten Substitutionsmuster auszuwählen sind.

Von den gebildeten Enantiomeren wird das jeweilige R-Enantiomer wegen seiner geringeren Teratogenität bevorzugt.

In den folgenden Tabellen ist gezeigt, daß die erfindungsgemäßen Verbindungen hinsichtlich der antiepileptischen Wirkung, der sedativen Wirkung und der teratogenen Wirkung bzw. der Exencephaliewirkung den bisher bekannten als Antiepileptikum in Frage kommenden Verbindungen überlegen sind.

Die antikonvulsiven, sedierenden und teratogenen Wirkungen der erfindungsgemäßen Verbindungen wurden nach den im folgenden beschriebenen Verfahren bestimmt.

Die antikonvulsive Wirkung der erfindungsgemäßen Carbonsäuren wurde gegenüber subcutan verabreichtem, konvulsiv wirkendem Pentetrazol (PTZ), in Anlehnung an den von Swinyard et al., Laboratory evaluation of antiepileptic drugs, Review of laboratory methods, Epilepsia **10**, 107-119 (1969) beschriebenen subkutanen Pentetrazol-Krampf-Test (PTZ-Test), bestimmt. Pro Substanz und Dosis werden Gruppen von 5 bis 8 Mäusen (NMRI oder Swiss albino) verwendet. Die Lösungen der Natriumsalze der Carbonsäuren werden den Mäusen i.p. injiziert (1,50 mMol/kg); 15 Minuten später wird eine Lösung von 65 mg PTZ in 10 ml physiologischer Natriumchlorid-Lösung/kg Körpergewicht subcutan in eine Hautfalte am Nacken appliziert. Die Tiere werden anschließend 30 Minuten lang beobachtet. Die Tiere, bei denen in diesem Zeitraum keine klonischen Spasmen auftreten, die mindestens 5 Sekunden anhalten, gelten im Sinne des PTZ-Tests als geschützt. PTZ allein bewirkt nach 6 bis 12 Minuten, sowohl in NMRI- als auch in Swiss albino-Mäusen klonische Spasmen, die länger als 5 Sekunden andauern. Diese klonischen Spasmen gehen nicht in tonische Krämpfe über.

Die Bestimmung der teratogenen Wirkung der erfindungsgemäßen Carbonsäuren wurde nach dem von Nau in Toxicol. Appl. Pharmacol. **80**, 243 - 250 (1985) für die Untersuchung von Valproinsäure und anderen Verbindung beschriebenen Exencephalie-Modell in der Maus durchgeführt. Weibliche NMRI-Mäuse werden zwischen 6.00 und 9.00 Uhr mit männlichen NMRI-Mäusen gepaart. Als Tag Null der Schwangerschaft gelten die ersten 24 Stunden nach der Konzeption. Die Lösung der Natriumsalze der Carbonsäuren werden den Mäusen am Tag 8 der Schwangerschaft zwischen 7.00 und 9.00 Uhr i.p. injiziert. Am Tag 18 der Schwangerschaft, zwischen 9.00 und 12.00 Uhr, werden die Tiere mit Diethylether eingeschläfert und anschließend wird der Uterus entnommen. Die Zahl der Implantationsstellen und die der Resorptionen und toten Feten (Embryoletalität) wird festgestellt. Jeder lebende Fetus wird gewogen und auf Exencephalie untersucht.

Die sedierende Wirkung (maternale Toxizität) der erfindungsgemäßen Carbonsäuren wurde mit Hilfe des Rotorod-Toxizitäts-Tests bestimmt, der von Dunham und Miya beschrieben wurde (The note on a simple apparatus for detecting neurological deficit in rats and mice, J.Am. Pharm. Assoc. **46**, 208-209 (1957). Die Lösungen der Natriumsalze der Carbonsäuren werden den Mäusen in einer Dosis von 1,50 mMol/kg i.p. injiziert. 15 Minuten später wird untersucht, ob die Tiere noch in der Lage sind, sich auf der sich mit 15 UpM drehenden Stange des Rotorod-Apparates (Rotorod, Ugobasile, Italien) 1 Minute lang zu halten. Diese Untersuchung wird für jede Substanz an 5 bis 7 Mäusen durchgeführt. Die Mäuse müssen sich, vor Verabreichung der Verbindungen, mindestens 5 Minuten lang auf der Stange des Rotorod-Apparates halten können.

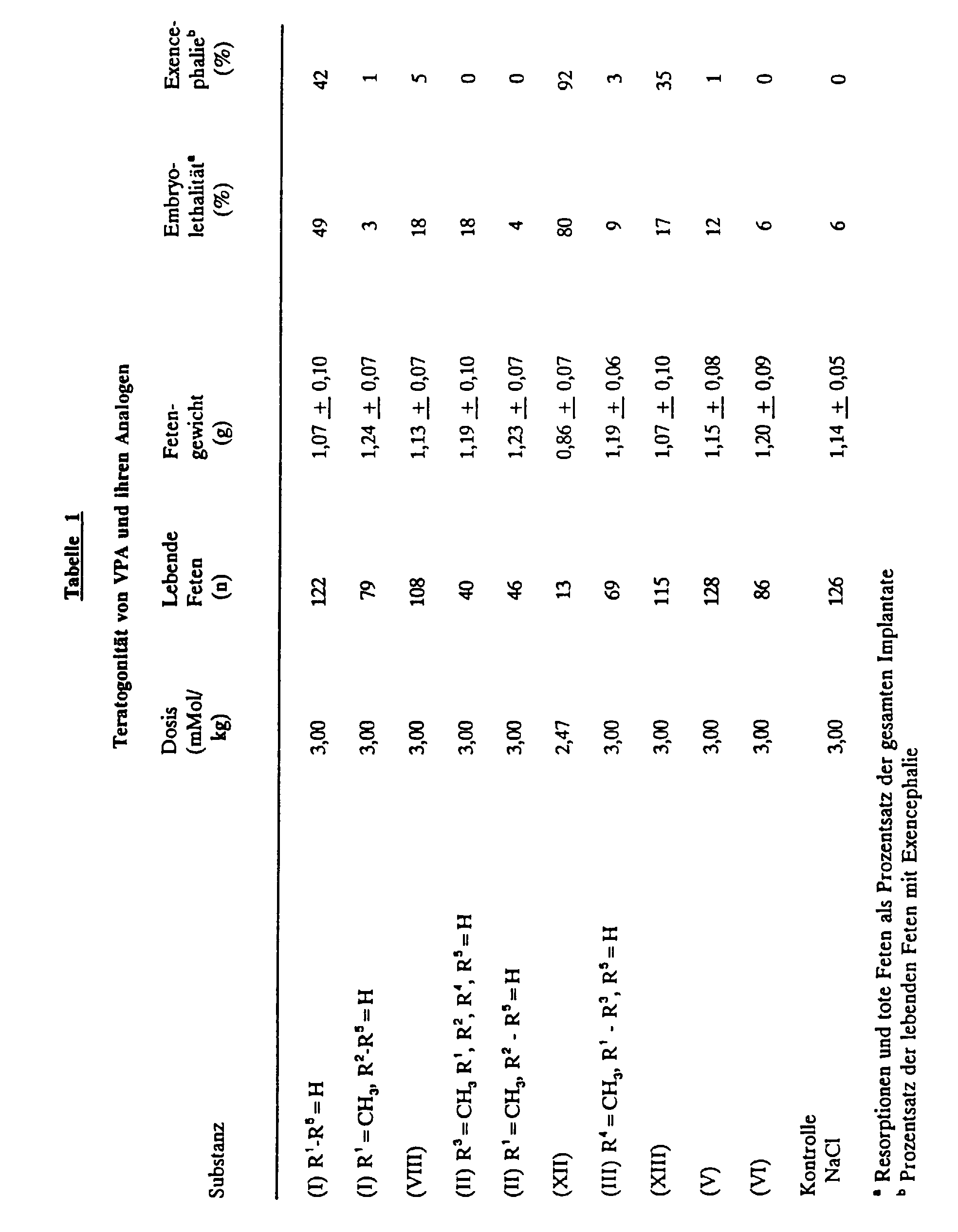

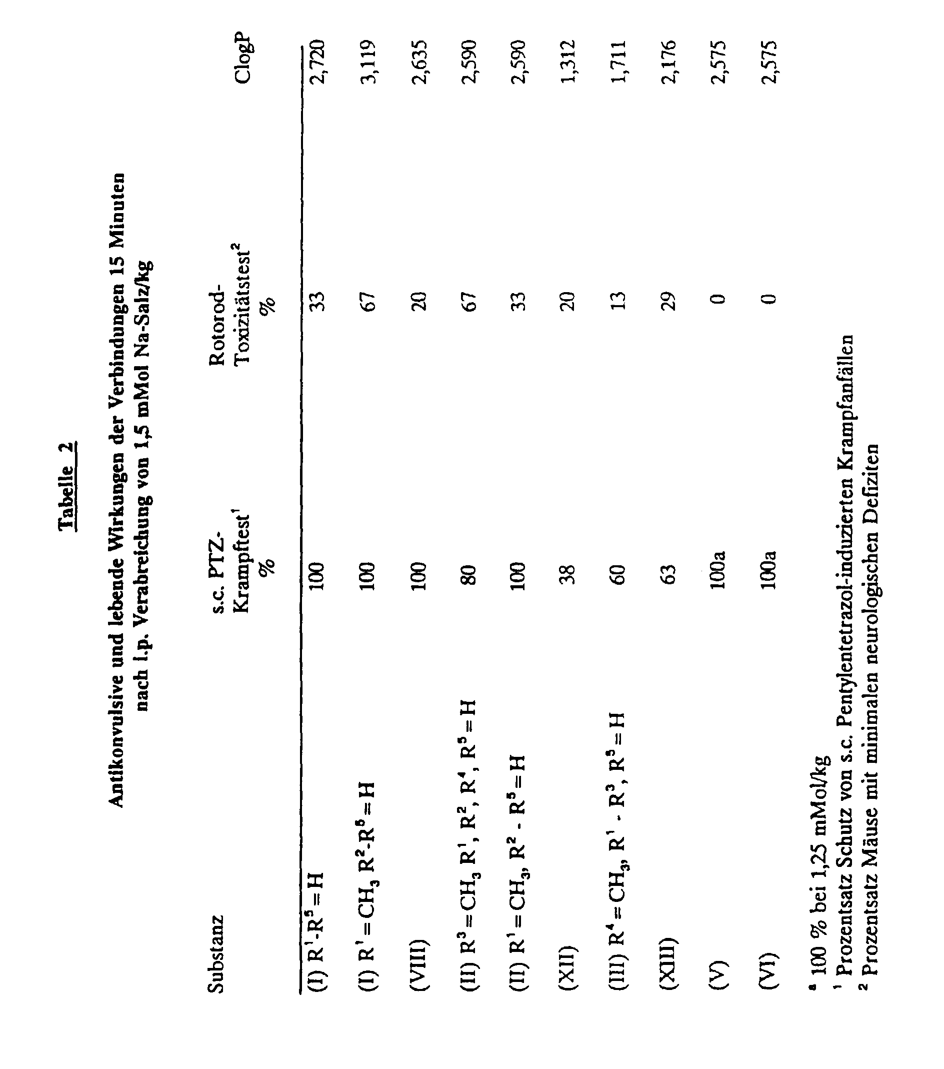

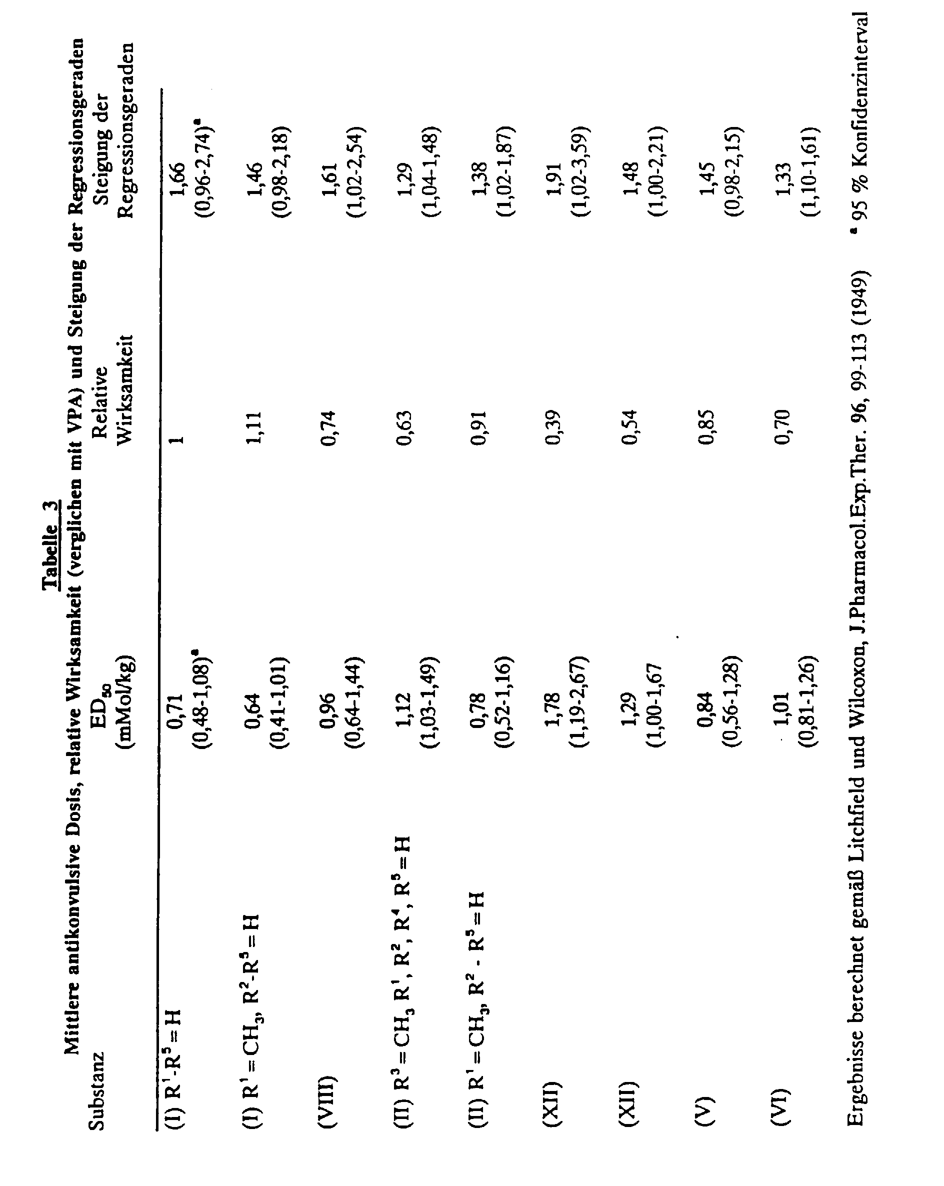

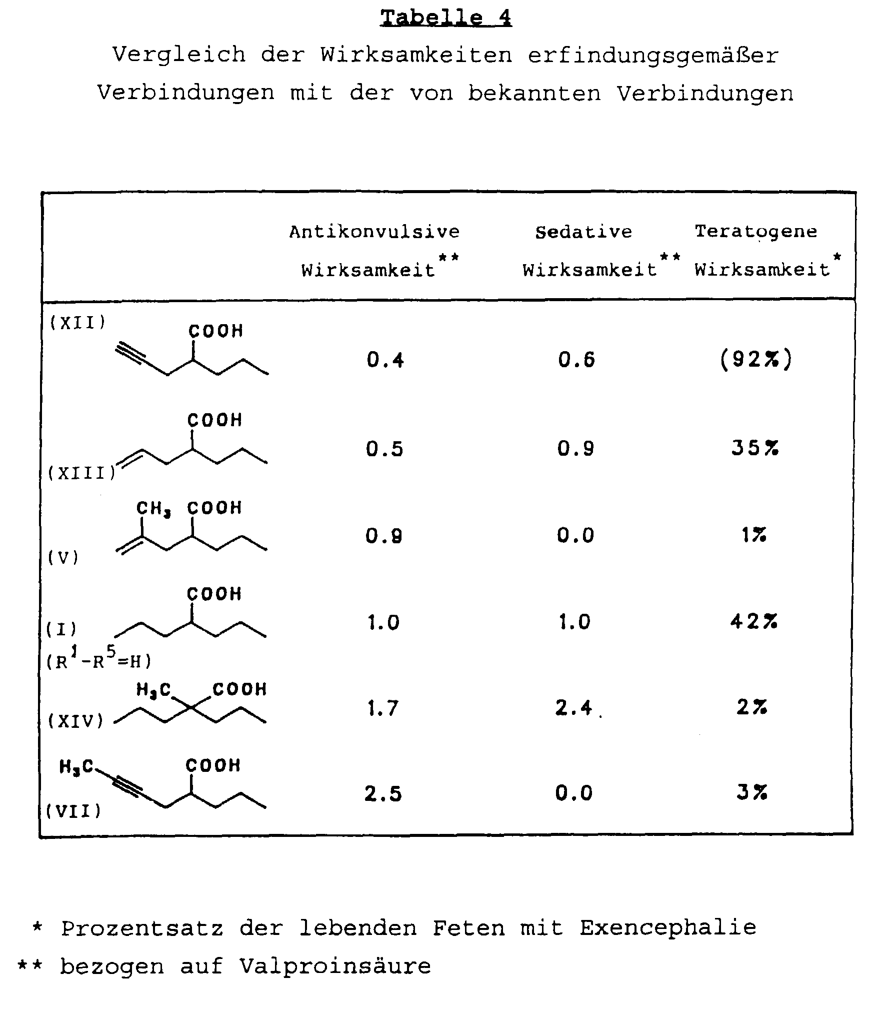

## Patentansprüche

1. Verbindungen der allgemeinen Formel 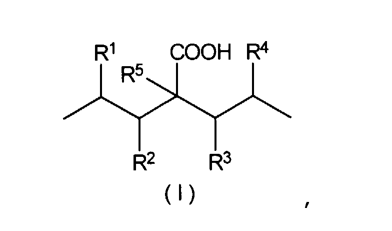 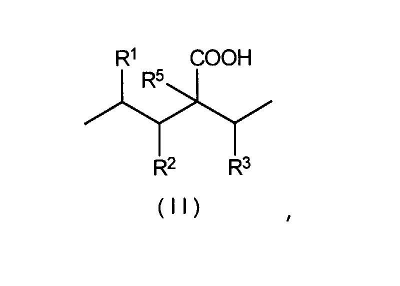 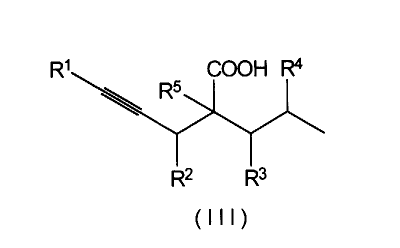 oder 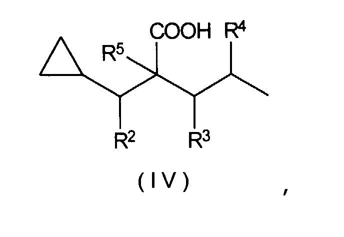 wobei die Verbindungen der Formel (I) und (II) auch ein- oder mehrfach ungesättigt sein können, in denen R¹, R², R³ und R⁴ unabhängig voneinander Wasserstoff oder eine C₁-C₆-Alkylgruppe sind, R⁵ Wasserstoff oder eine C₁-C₂-Alkylgruppe ist und bei den Verbindungen der Formeln (I) und (II) mindestens einer der Reste R¹ bis R⁴ von Wasserstoff verschieden ist, wobei die Verbindungen der Formel (I), wenn am Kohlenstoffatom in der 2-Position ein Asymmetriezentrum vorhanden ist, in Form ihres Racemats, ihrer reinen Enantiomere oder eines vom Racemat verschiedenen Gemisches ihrer Enantiomere vorliegen und die Verbindungen der Formeln (II), (III) und (IV) in Form ihrer reinen Enantiomere oder eines vom Racemat verschiedenen Gemisches ihrer Enantiomere vorliegen, wobei die Verbindungen der Formel (III), wenn sie an mindestens an einer anderen als der 5-Position alkyliert sind, und die Verbindungen der Formel (IV), wenn sie mindestens einfach alkyliert sind, auch in Form ihres Racemats vorliegen, sowie pharmazeutisch verträgliche Salze derselben.

2. Verbindungen nach Anspruch 1, dadurch gekennzeichnet, daß die Reste R¹ bis R⁵ Wasserstoff oder Methylgruppen sind.

3. Verbindungen nach Anspruch 1 oder 2, dadurch gekennzeichnet, daß die Verbindungen der Formeln (I) und (II) am Kohlenstoffatom in der 4-Position und/oder 2'-Position ungesättigt sind.

4. Verbindungen nach einem der Ansprüche 1 bis 3, dadurch gekennzeichnet, daß sie die allgemeine Formel 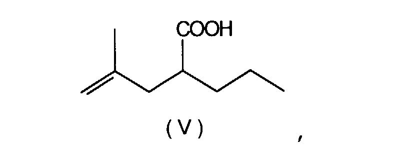 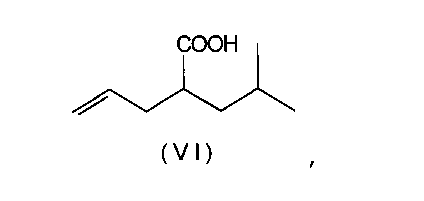 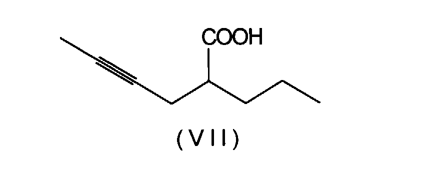 oder 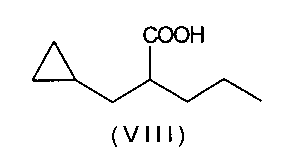 aufweisen.

5. Verbindungen der allgemeinen Formel 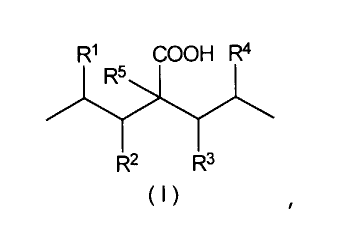 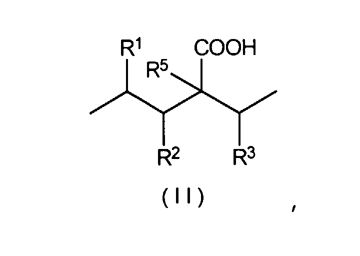 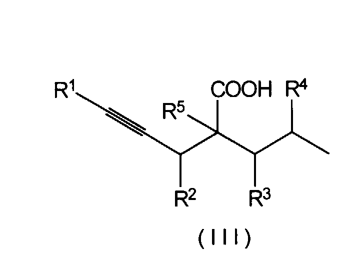 oder 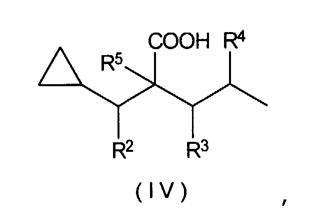 wobei die Verbindungen der Formeln (I) und (II) auch ein- oder mehrfach ungesättigt sein können, in denen R¹, R², R³ und R⁴ jeweils unabhängig voneinander Wasserstoff oder eine C₁-C₆-Alkylgruppe sind, R⁵ Wasserstoff oder eine C₁-C₂-Alkylgruppe ist und bei den Verbindungen der Formeln (I) und (II) mindestens einer der Reste R¹ bis R⁴ von Wasserstoff verschieden ist, wobei die Verbindung der Formel (I), wenn am Kohlenstoffatom in der 2-Position ein Asymmetriezentrum vorhanden ist, und die Verbindung der Formeln (II), (III) und (IV) in Form ihres Racemats, ihrer reinen Enantiomere oder eines vom Racemat verschiedenen Gemisches ihrer Enantiomere vorliegen, oder pharmzeutisch verträgliche Salze derselben, als Arzneimittel.

6. Verbindungen nach Anspruch 5, dadurch gekennzeichnet, daß die Reste R¹ bis R⁵ Wasserstoff oder Methylgruppen sind, als Arzneimittel.

7. Verbindungen nach Anspruch 5 oder 6, dadurch gekennzeichnet, daß die Verbindungen außerdem am Kohlenstoffatom in der 4-Position und/oder 2'-Position ungesättigt sind, als Arzneimittel.

8. Verbindungen nach einem der Ansprüche 5 bis 7, dadurch gekennzeichnet, daß die Verbindungen die allgemeine Formel 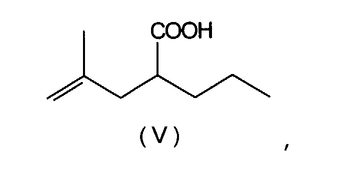 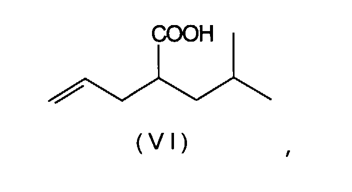 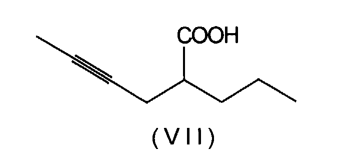 oder 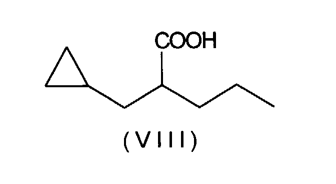 aufweisen, als Arzneimittel.

9. Verwendung einer Verbindung der allgemeinen Formel 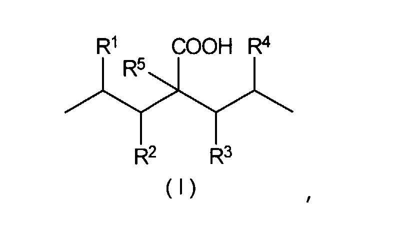 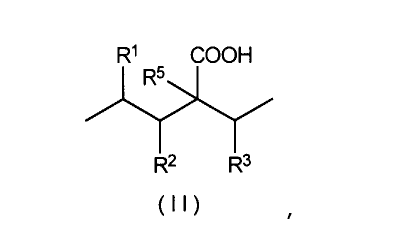 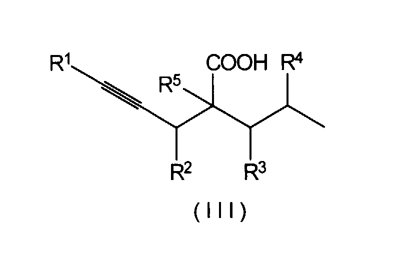 oder 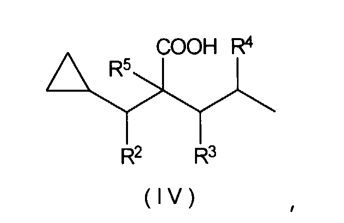 wobei die Verbindungen der allgemeinen Formeln (I) und (II) auch ein- oder mehrfach ungesättigt sein können, in denen R¹, R², R³ und R⁴ unabhängig voneinander Wasserstoff oder eine C₁-C₆-Alkylgruppe sind, R⁵ Wasserstoff oder eine C₁-C₂-Alkylgruppe ist und bei den Verbindungen der allgemeinen Formeln (I) und (II) mindestens einer der Reste R¹ bis R⁴ von Wasserstoff verschieden ist, wobei die Verbindung der allgemeinen Formel (I), wenn am Kohlenstoffatom in der 2-Position ein Asymmetriezentrum vorhanden ist, und die Verbindung der allgemeinen Formeln (II), (III) und (IV) in Form ihres Racemats, ihrer reinen Enantiomere oder eines vom Racemat verschiedenen Gemisches ihrer Enantiomere vorliegen, oder pharmazeutische verträgliche Salze derselben zur Herstellung eines Antiepileptikums.

10. Verwendung einer Verbindung nach Anspruch 9, dadurch gekennzeichnet, daß die Reste R¹ bis R⁵ Wasserstoff oder Methylgruppen sind, zur Herstellung eines Antiepileptikums.

11. Verwendung einer Verbindung nach Anspruch 9 oder 10, dadurch gekennzeichnet, daß die Verbindung außerdem am Kohlenstoffatom in der 4-Position und/oder 2'-Position ungestättigt ist, zur Herstellung eines Antiepileptikums.

12. Verwendung einer Verbindung nach einem der Ansprüche 9 bis 11, dadurch gekennzeichnet, daß die Verbindung die allgemeine Formel 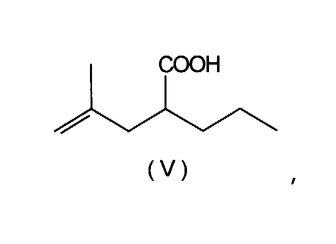 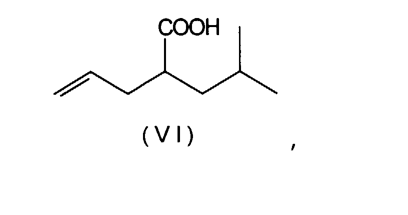 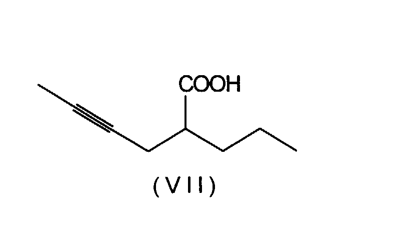 oder 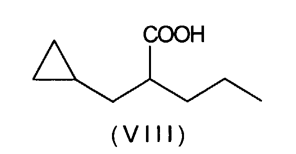 aufweist, zur Herstellung eines Antiepileptikums.

13. Verfahren zur Herstellung der Verbindungen der Formeln (I) bis (IV), dadurch gekennzeichnet, daß ein 2-Alkylmalonsäuredialkylester der Formel 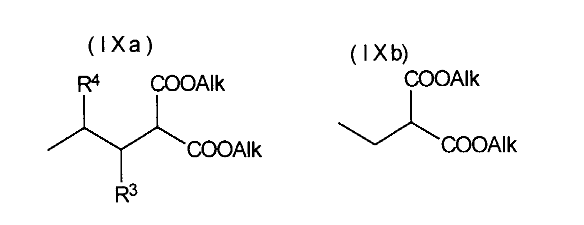 mit einer Base deprotoniert und einem Alkylierungsmittel der Formel 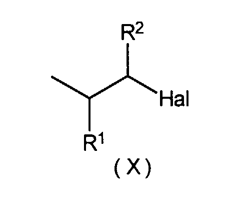 Alk = Methyl, Ethyl
am Kohlenstoffatom in der 2-Position alkyliert wird, der dialkylierte Malonsäurediethylester der Formel 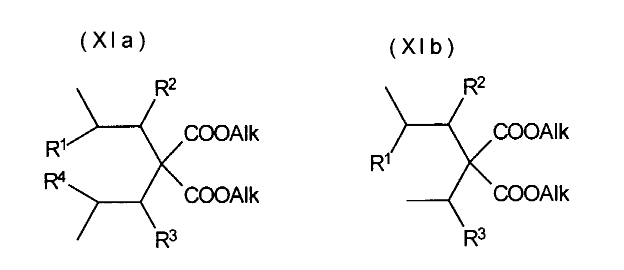 alkalisch verseift und unter Erhitzen decarboxyliert wird.

14. Verfahren zur Herstellung der Enantiomere der Verbindungen der Formeln (I) bis (IV), dadurch gekennzeichnet, daß aus dem Oxazolidinon (XV) oder (XVI) unter Verwendung von Valeroylchlorid oder Butanoylchlorid das entsprechende N-acylierte Oxazolidinon gebildet wird, welches nach Erzeugung des Anions mit dem jeweiligen Alkylierungsmittel (X) alkyliert wird und sich nach Abspaltung des Oxazolidinons das jeweilige gewünschte Enantiomer ergibt.

## Claims

1. Compounds of the general formula 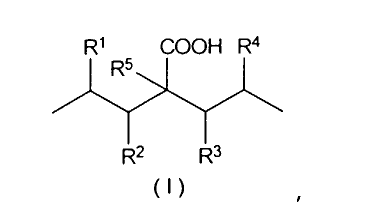 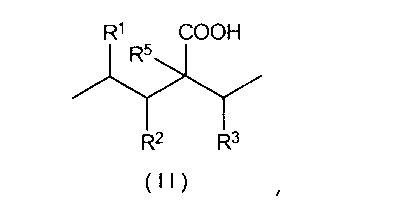 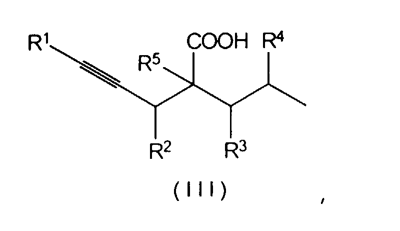 or 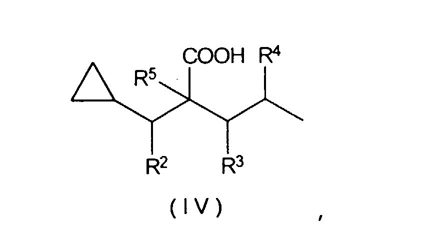 where the compounds of the formulae (I) and (II) can 3 also be mono- or polyunsaturated, in which R¹, R², R³ and R⁴ are, independently of one another, hydrogen or a C₁-C₆-alkyl group, R⁵ is hydrogen or a C₁-C₂-alkyl group, and at least one of the radicals R¹ to R⁴ in the compounds of the formulae (I) and (II) is different from hydrogen, where the compounds of the formula (I) are, when a center of asymmetry is present on the carbon atom in position 2, in the form of their racemate, their pure enantiomers or a mixture of their enantiomers which differs from the racemate, and the compounds of the formulae (II), (III) and (IV) are in the form of their pure enantiomers or a mixture of their enantiomers which differs from the racemate, where the compounds of the formula (III) are, when they are alkylated in at least one position different from position 5, and the compounds of the formula (IV) are, when they are at least alkylated once, also in the form of their racemate, as well as pharmaceutically compatible salts thereof.

2. Compounds as claimed in claim 1, characterized in that the radicals R¹ to R⁵ are hydrogen or methyl groups.

3. Compounds as claimed in claim 1 or 2, characterized in that the compounds of the formular (I) and (II) are unsaturated at the carbon atom in position 4 and/or position 2'.

4. Compounds as claimed in any of claims 1 to 3, characterized in that they have the formula 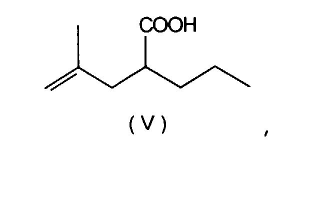 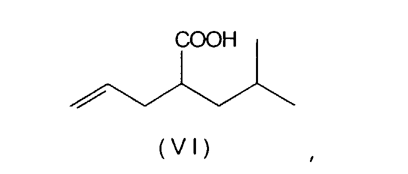 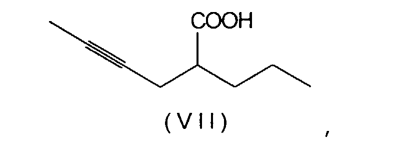 or

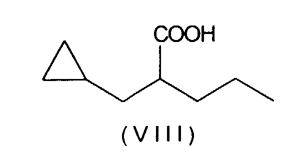

5. Compounds for the use as medicaments of the general formulae 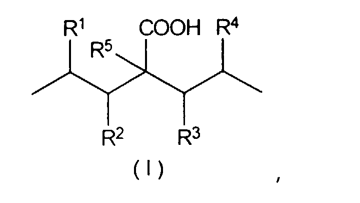 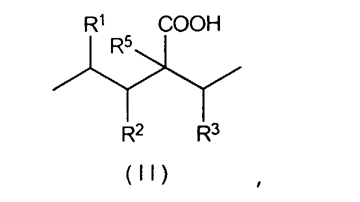 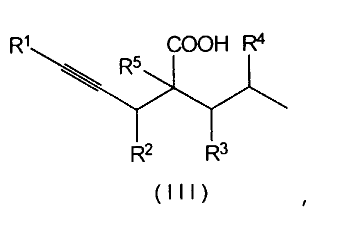 or 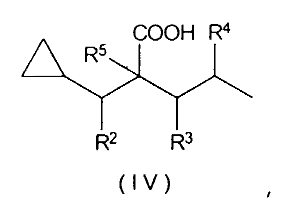 wherein the compounds of the formulae (I) and (II) can also be mono- or polyunsaturated, in which R¹, R², R³ and R⁴ are, independently of one another, hydrogen or a C₁-C₆-alkyl group, R⁵ is hydrogen or a C₁-C₂-alkyl group, and at least one of the radicals R¹ to R⁴ in the compounds of the formulae (I) and (II) is different from hydrogen, where the compound of the formula (I) is, in the case where a center of asymmetry is present on the carbon atom in position 2, and the compounds of the formulae (II), (III) and (IV) are in the form of their racemate, their pure enantiomers or a mixture of their enantiomers which differs from the racemate, or pharmaceutically compatible salts thereof.

6. Compounds for the use as medicaments as claimed in claim 5, characterized in that the radicals R¹ to R⁵ are hydrogen or methyl groups.

7. Compounds for the use as medicaments as claimed in claim 5 or 6 , characterized in that the compound is additionally unsaturated at the carbon atom in position 4 and/or position 2'.

8. Compounds for the use as medicaments as claimed in any of claim 5 to 7, characterized in that the compound has the formula 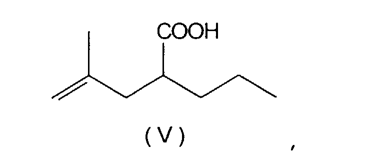 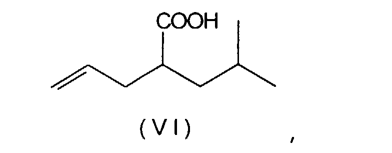 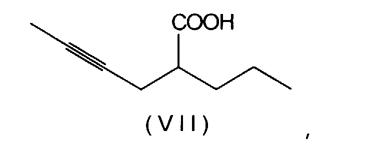 or

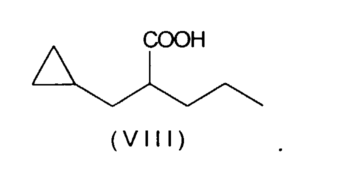

9. The use of a compound of the general formula 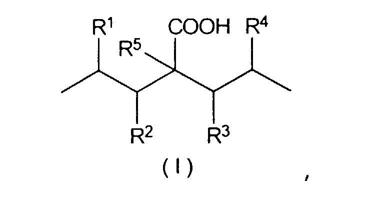 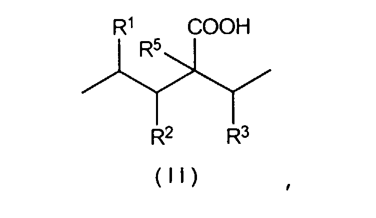 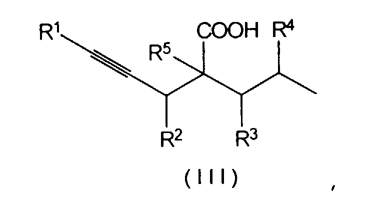 or 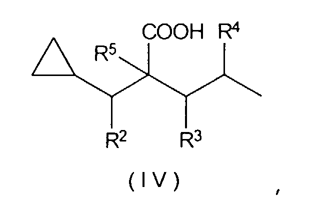 wherein the compounds of the formulae (I) and (II) can also be mono- or polyunsaturated, in which R¹, R², R³ and R⁴ are each, independently of one another, hydrogen or a C₁-C₆-alkyl group, R⁵ is hydrogen or a C₁-C₂-alkyl group, and at least one of the radicals R¹ to R⁴ in the compounds of the formulae (I) and (II) is different from hydrogen, where the compound of the formula (I), in the case where a center of asymmetry is present on the carbon atom in position 2, and the compounds of the formulae (II), (III) and (IV) are in the form of their racemate, their pure enantiomers or a mixture of their enantiomers which differs from the racemate, or pharmaceutically compatible salts thereof, for production of an antiepileptic.

10. Use of a compound as claimed in claim 9, characterized in that the radicals R¹ to R⁵ are hydrogen or methyl groups, for production of an antiepileptic.

11. Use of a compound as claimed in claim 9 or 10, characterized in that the compound is additionally unsaturated at the carbon atom in position 4 and/or 2', for production of an antiepileptic.

12. use of a compound as claimed in any of claims 9 to 11, wherein the compound has the general formula 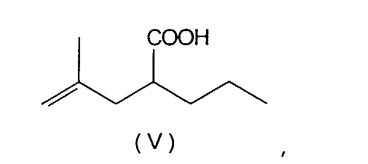 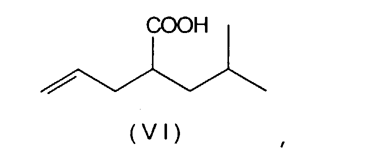 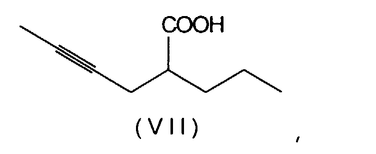 or 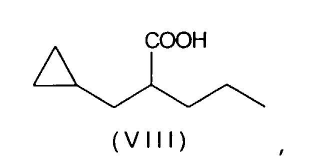 for production of an antiepileptic.

13. Processes for preparing compounds of the formulae (I) to (IV), which comprises a dialkyl 2-alkylmalonate of the formula 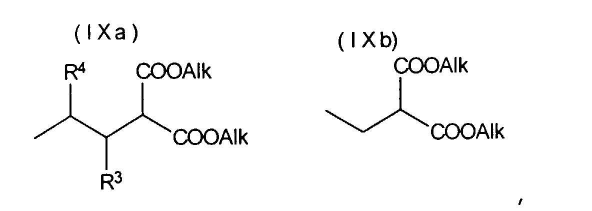 Alk = methyl, ethyl
being deprotonated with a base and being alkylated on the carbon atom in position 2 with an alkylating agent of the formula 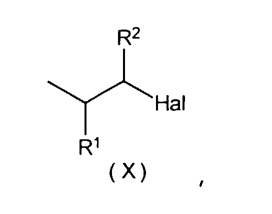 and the dialkylated dialkyl malonate of the formula 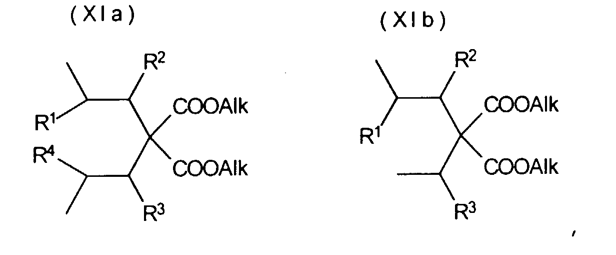 undergoing alkaline hydrolysis and decarboxylation by heating.

14. Processes for preparing the enantiomers of the compounds of the formulae (I) to (IV), which comprises forming from the oxazolidinone (XV) or (XVI), using valeroyl chloride or butanyl chloride, the corresponding N-acetylated oxazolidinone, which, after generation of the anion, is alkylated with the particular alkylating agent (X) and yields the required enantiomer in each case after elimination of the oxazolidinone.

## Revendications

1. Composés de formules générales 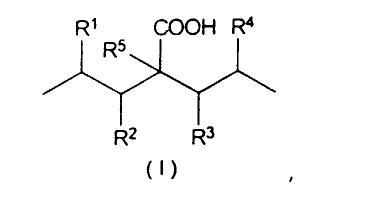 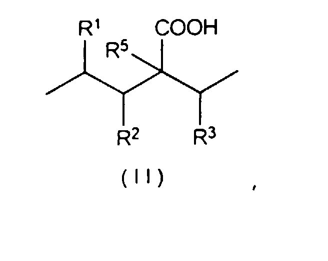 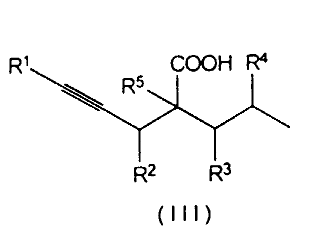 ou 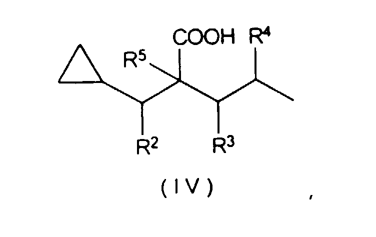
dans lesquels les composés des formules I et II peuvent être également mono ou pluri-insaturés, et R¹, R², R³ et R⁴ dans ces formules sont indépendamment l'un de l'autre l'hydrogène ou un groupe C₁-C₆ alkyle, R⁵ l'hydrogène ou un groupe C₁-C₂ alkyle, et au moins un des groupes R¹ à R⁴ dans les formules I et II, est différent de l'hydrogène,
dans lesquels les composés de formule I lorsqu'un centre d'asymétrie est présent sur l'atome de carbone en position 2, sont présents sous forme de leur racémique, leurs énantiomères purs ou un mélange de leurs énantiomères différent de leur racémique et les composés des formules II, III et IV sont présents sous forme de leur énantiomères purs ou d'un mélange de leurs énantiomères différent du racémique, et
dans lesquels les composés de formule III lorsqu'ils sont alkylés en au moins une position autre que la position 5, et les composés de formule IV lorsqu'ils sont au moins monoalkylés, sont présents également sous forme de leur racémique,
ainsi que les sels pharmaceutiquement acceptables de ces composés.

2. Composés selon la revendication 1, caractérisés en ce que les groupes R¹ à R⁵ sont l'hydrogène ou le groupe méthyle.

3. Composés selon la revendication 1 ou 2, caractérisés en ce que les composés de formule I et II sont insaturés sur l'atome de carbone en position 4 et/ou 2'.

4. Composés selon l'une des revendications 1 à 3, caractérisés en ce qu'ils répondent aux formules générales 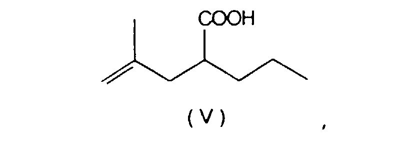 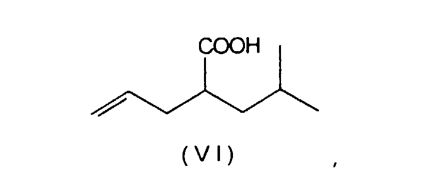 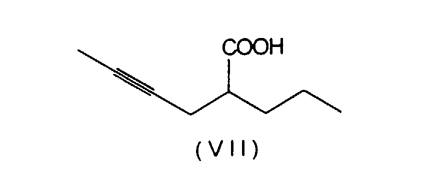 ou

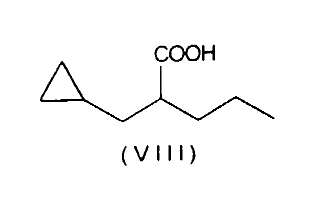

5. Composés de formules générales 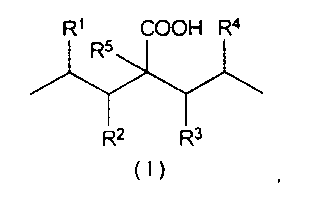 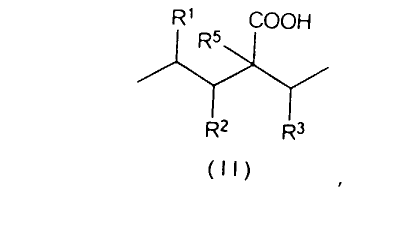 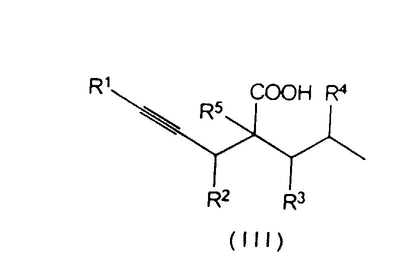 ou 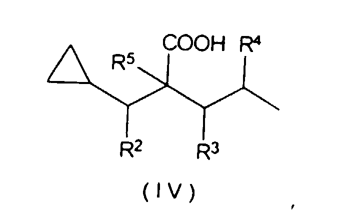
dans lesquels les composés des formules I et II peuvent également être mono ou pluri-insaturés, et R¹, R², R³ et R⁴ dans ces formules étant indépendamment l'un de l'autre l'hydrogène ou un groupe C₁-C₆ alkyle, R⁵ l'hydrogène ou un groupe C₁-C₂ alkyle, et, dans les composés de formule I et II, au moins un des groupes R¹ à R⁴ sont différents de l'hydrogène,
dans lesquels le composé de formule I, lorsqu'un centre d'asymétrie est présent sur l'atome de carbone en position 2, et le composé de formules II, III et IV sont présents sous la forme de leur racémique, leurs énantiomères purs ou un mélange de leurs énantiomères différent du racémique,
ainsi que les sels pharmaceutiquement acceptables en tant que médicaments.

6. Composés selon la revendication 5, caractérisés en ce que les groupes R¹ à R⁵ sont l'hydrogène ou le groupe méthyle, en tant que médicaments.

7. Composés selon la revendication 5 ou 6, caractérisés en ce que les composés sont en outre insaturés sur l'atome de carbone en position 4 et/ou 2', en tant que médicaments.

8. Composés selon l'une quelconque des revendications 5 à 7, caractérisés en ce que les composés présentent les formules générales 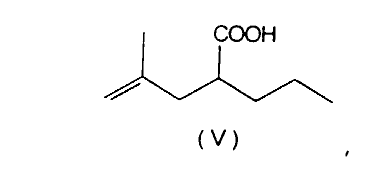 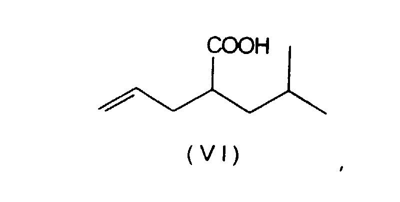 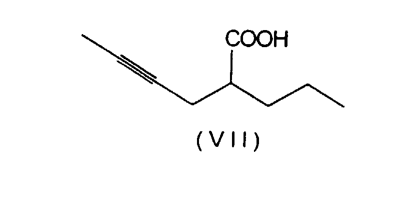 ou 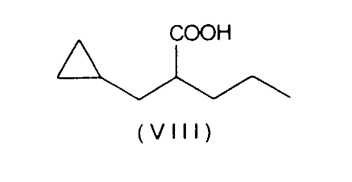 en tant que médicaments.

9. Utilisation d'un composé de formules générales 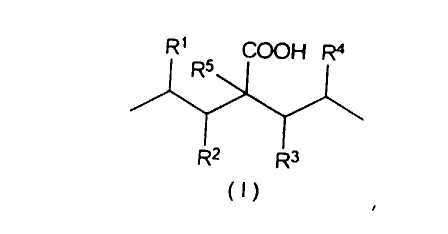 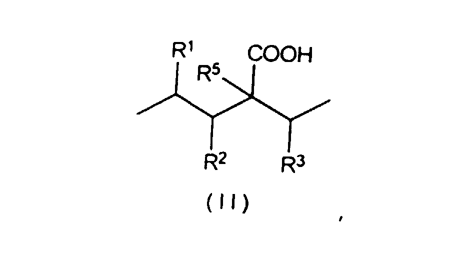 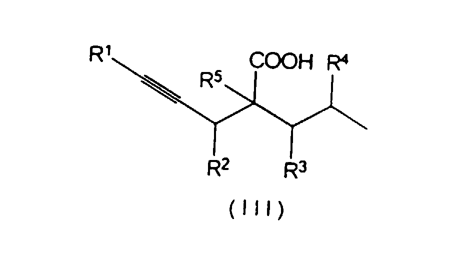 ou 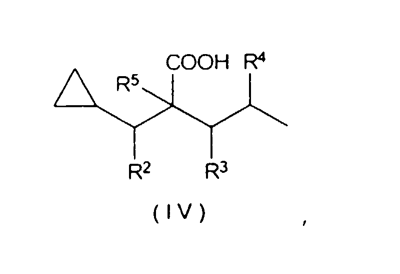
dans lesquels les composés des formules générales I et II peuvent être également mono ou pluri-insaturés, et les composés R¹, R², R³ et R⁴ sont indépendamment l'un de l'autre l'hydrogène ou un groupe C₁-C₆ alkyle, R⁵ l'hydrogène ou un groupe C₁-C₂ alkyle, et au moins un des groupes R¹ à R⁴ dans les composés de formule I ou II est différent de l'hydrogène,
dans lesquels le composé de formule générale (I) lorsqu'un centre d'asymétrie est présent sur l'atome de carbone en position 2, et le composé de formule générale II, III et IV sont présents sous la forme de leur racémique, leurs énantiomères purs ou un mélange de leurs énantiomères différent du racémique, ou les sels pharmaceutiquement acceptables de ces composés pour la préparation d'un médicament anti-épileptique.

10. Utilisation d'un composé selon la revendication 9, caractérisée en ce que les groupes R¹ à R⁵ sont l'hydrogène ou le groupe méthyle, pour la préparation d'un médicament anti-épileptique.

11. Utilisation d'un composé selon la revendication 9 ou 10, caractérisée en ce que le composé est en outre insaturé sur l'atome de carbone en position 4 et/ou 2', pour la préparation d'un médicament anti-épileptique.

12. Utilisation d'un composé selon la revendication 9 à 11, caractérisée en ce que le composé est représenté par les formules générales 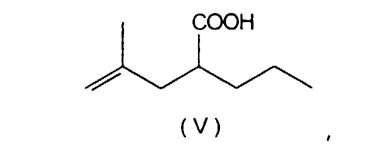 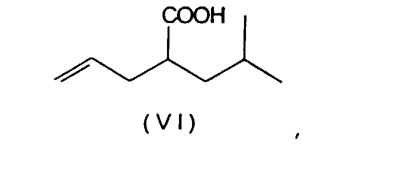 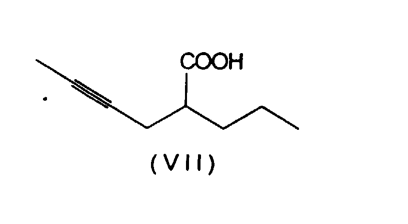 ou 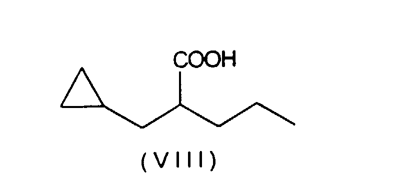 pour la préparation d'un médicament antiépileptique.

13. Procédé de préparation d'un composé de formule (I) à (IV) caractérisé en ce que un esterdialkylique de l'acide 2-alkyl-malonique de formule 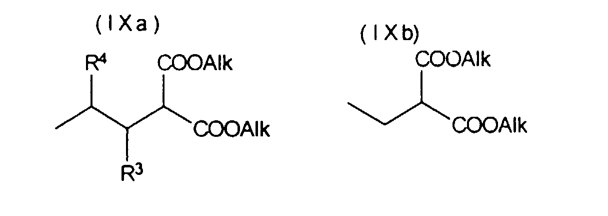 est déprotoné avec une base et alkylé avec un agent d'alkylation de formule 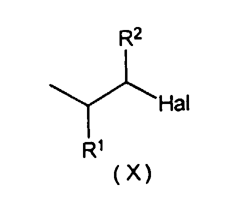 Alk = méthyle, éthyle
sur l'atome de carbone en position 2, et ledit diéthylester de l'acide dialkyl-malonique de formule 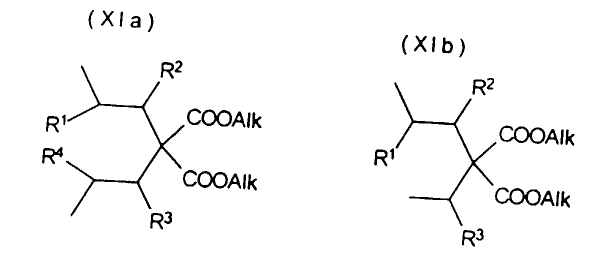 est saponifié en milieu alcalin et décarboxylé par chauffage.

14. Procédé de préparation des énantiomères des composés de formules I à IV, caractérisé en ce que, à partir de l'oxazolidinone V ou XVI l'oxazolidinone N-acylée correspondant est obtenue par utilisation de chlorure de valeroyle ou du chlorure de butanoyle, laquelle oxazolidinone N-acylée est, après obtention de l'anion, alkylée avec l'agent d'alkylation X correspondant, et, après séparation de l'oxazolidinone, l'énantiomère correspondant souhaité est obtenu.
